# EUROPEAN PATENT APPLICATION

(11) **EP 4 603 085 A1**
(43) Date of publication of application: **20.08.2025**
(21) Application number: 23876670.3
(22) Date of filing: 10.10.2023
(51) Int. Cl.: A61K 31/05, A61P 17/02

(54) **USE OF STILBENE DERIVATIVE IN PREVENTION AND/OR TREATMENT OF ULCERS**

(30) Priority: 10.10.2022 CN 202211236827
(71) Applicant: Thederma Shanghai Co., Ltd., Shanghai 201203 (CN)
(72) Inventor: JIA, Jianmin, Shanghai 201203 (CN); CAI, Yaxian, Shanghai 201203 (CN); MA, Zhilong, Shanghai 201203 (CN); CHEN, Ming, Shanghai 201203 (CN); OUYANG, Xiaowen, Shanghai 201203 (CN); JING, Xiangyan, Shanghai 201203 (CN)
(74) Representative: Gille Hrabal Partnerschaftsgesellschaft mbB Patentanwälte
(86) International application number: PCT/CN2023/123650
(87) International publication number: WO 2024/078468

(57) **Abstract**

The present disclosure relates to use of a stilbene derivative in the prevention and/or treatment of ulcers. A compound of formula (I) or a pharmaceutically acceptable salt thereof can effectively prevent or treat ulcers, and in particular, has a curative effect on oral ulcers.

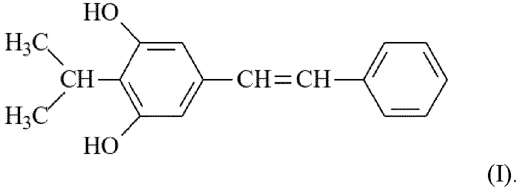

## Description

The present application claims priority to Chinese Patent Application No. 202211236827.3, filed to China National Intellectual Property Administration on October 10, 2022 and entitled "USE OF STILBENE DERIVATIVE IN PREVENTION AND/OR TREATMENT OF ULCERS", which is incorporated herein by reference in its entirety.

### TECHNICAL FIELD

The present disclosure relates to use of a stilbene derivative in the prevention and/or treatment of an ulcer and to the field of pharmaceutics.

### BACKGROUND

Ulcers are ulcerations or deletions of mucous membranes or skins of human bodies, are frequently found in gastrointestinal tracts, oral cavities, genital organs, and other parts clinically, and have complex etiology.

An oral ulcer, commonly called "aphthous ulcer", is a common ulcerative injury disorder occurring in oral mucosa, which is usually found in the inner lip, tongue, lingual undersurface, buccal mucosa, vestibular sulcus, soft palate, and other parts, and has relatively high morbidity at present. The oral ulcer includes a recurrent oral ulcer, a traumatic oral ulcer, and an oral ulcer associated with a disease. Among them, the recurrent oral ulcer is also called a recurrent aphthous ulcer or a frequent oral ulcer. Guidelines for Diagnosis and Treatment of Recurrent Aphthous Ulcer published in 2012 in China states that 10%-25% of people suffer from recurrent oral ulcers, and the age of onset for recurrent oral ulcers is 10-30 years old; the traumatic oral ulcer includes oral ulcers caused by a mechanical injury (such as oral mucosal injury caused by hard substances such as fishbone and sandstone in food, toothbrush, dental calculus deposited on the surface of teeth, etc.), a chemical burn (such as local oral mucosal injury caused by irritant drugs such as analgesic buccal tablets (e.g., aspirin), silver nitrate, iodine tincture, etc.), and cold and hot stimulation (such as mucosal burns caused by excessively high water temperature, excessively scalding food, etc., or mucosal frostbite caused by improper low-temperature treatment operation); the oral ulcer associated with a disease includes oral ulcers associated with diseases such as Behcet's disease, Reiter's syndrome, a tumor, etc.

At present, the pathogenic cause of the oral ulcers is not clear, and possible inducing factors include genetic factors, dietary factors, immune factors, mental factors, traumatic factors, or the like. The drug treatment for oral ulcers preferably employs local treatment, including the administration of analgesic drugs (such as lidocaine, benzocaine, and benzydamine), anti-inflammatory drugs (such as chlorhexidine and Cydiodine), healing-promoting drugs (such as Bingpengsan, Watermelon Frost, and recombinant human epidermal growth factors), Kangfuxin Ye, and other drugs. For patients with severe oral ulcers or high recurrence frequency, systemic administration including the administration of immune formulations, vitamins, or the like can be used.

At present, there is still a need to develop a new medicament having a good therapeutic effect on ulcers, especially oral ulcers.

### SUMMARY

In order to solve the technical problems described above, the present disclosure provides use of at least one selected from a compound represented by formula (I) below and a pharmaceutically acceptable salt thereof for manufacturing a medicament for the prevention and/or treatment of an ulcer:

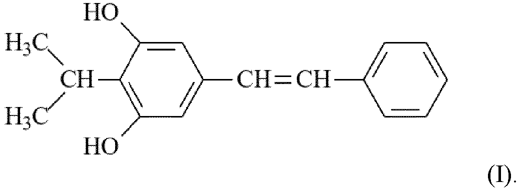

According to an embodiment of the present disclosure, the medicament is a pharmaceutical composition. Preferably, the pharmaceutical composition comprises at least one of the compound represented by formula (I) and the pharmaceutically acceptable salt thereof described above.

The present disclosure further provides a pharmaceutical composition comprising at least one of the compound represented by formula (I) and the pharmaceutically acceptable salt thereof described above, wherein the pharmaceutical composition is used for preventing and/or treating an ulcer. According to an embodiment of the present disclosure, the pharmaceutical composition comprises a therapeutically effective amount of at least one of the compound represented by formula (I) and the pharmaceutically acceptable salt thereof.

The present disclosure further provides use of the compound represented by formula (I), the pharmaceutically acceptable salt of the compound represented by formula (I), the medicament, or the pharmaceutical composition for preventing and/or treating an ulcer.

The present disclosure further provides the compound represented by formula (I), the pharmaceutically acceptable salt of the compound represented by formula (I), the medicament, or the pharmaceutical composition for use in preventing and/or treating an ulcer.

The present disclosure further provides a method for preventing and/or treating an ulcer, which comprises administering to a patient an effective amount or a therapeutically effective amount of at least one of the compound represented by formula (I) and the pharmaceutically acceptable salt thereof described above.

According to an embodiment of the present disclosure, the ulcer may be selected from one or more of a pressure ulcer (also known as a decubitus ulcer), a genital ulcer, ulcerative dermatitis, an anal ulcer, a rectal ulcer, a foot ulcer, a diabetic foot ulcer, a corneal ulcer, an oral ulcer, an aphthous ulcer (also known as a canker sore), a peptic ulcer, a venous ulcer, a stress ulcer (an ulcer located proximal to the stomach and duodenum), ulcerative sarcoidosis, ulcerative lichen planus, ulcerative colitis, and ulcerative disposition.

According to an embodiment of the present disclosure, the oral ulcer is selected from a recurrent oral ulcer, a traumatic oral ulcer, and an oral ulcer associated with or caused by a disease.

In the context of this specification, the recurrent oral ulcer is also called a recurrent aphthous ulcer or a frequent oral ulcer.

According to an embodiment of the present disclosure, the traumatic oral ulcer includes oral ulcers caused by a mechanical injury (such as oral mucosal injury caused by hard substances such as fishbone and sandstone in food, toothbrush, dental calculus deposited on the surface of teeth, etc.), a chemical burn (such as local oral mucosal injury caused by irritant drugs such as analgesic buccal tablets (e.g., aspirin), silver nitrate, iodine tincture, etc.), and cold and hot stimulation (such as mucosal burns caused by excessively high water temperature, excessively scalding food, etc., or mucosal frostbite caused by improper low-temperature treatment operation).

According to an embodiment of the present disclosure, the oral ulcer associated with or caused by a disease includes oral ulcers associated with or caused by diseases such as Behcet's disease, Reiter's syndrome, a tumor, etc.

According to an exemplary embodiment of the present disclosure, the medicament is used for preventing and/or treating at least one of the recurrent oral ulcer and the traumatic oral ulcer.

According to an embodiment of the present disclosure, the compound represented by formula (I) may be an E or Z isomer, preferably an E isomer represented by formula (I-1) below:

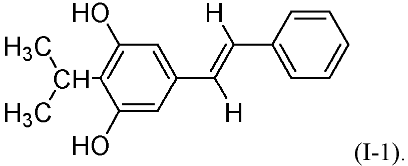

According to a preferred embodiment of the present disclosure, the compound represented by formula (I) is tapinarof.

According to an embodiment of the present disclosure, the pharmaceutically acceptable salt may be selected from addition salts of the compound represented by formula (I) with pharmaceutically acceptable bases.

Preferably, in the medicament or the pharmaceutical composition, only the compound represented by formula (I) is used as an active ingredient, and also, preferably, only the E isomer represented by formula (I-1) is used as the active ingredient.

According to an embodiment of the present disclosure, the daily administered dose of the medicament or the pharmaceutical composition is a therapeutically effective amount, e.g., 0.01-1 mg/kg/d, such as 0.02-0.4 mg/kg/d, exemplarily 0.05 mg/kg/d, 0.06 mg/kg/d, 0.07 mg/kg/d, 0.08 mg/kg/d, 0.09 mg/kg/d, 0.1 mg/kg/d, 0.12 mg/kg/d, 0.15 mg/kg/d, 0.2 mg/kg/d, 0.3 mg/kg/d, 0.4 mg/kg, 0.5 mg/kg, 0.6 mg/kg/d, 0.7 mg/kg/d, 0.8 mg/kg/d, 0.9 mg/kg/d, or 1 mg/kg/d, based on the compound represented by formula (I).

According to an embodiment of the present disclosure, the medicament or the pharmaceutical composition is preferably a pharmaceutical formulation. More preferably, the pharmaceutical composition is a single-dose formulation, wherein in the single-dose formulation, the content of the compound represented by formula (I) and the pharmaceutically acceptable salt thereof is a therapeutically effective amount, e.g., 1-10 mg, such as 1 mg, 2 mg, 3 mg, 4 mg, 5 mg, 6 mg, 7 mg, 8 mg, 9 mg, or 10 mg, based on the compound represented by formula (I).

According to an embodiment of the present disclosure, the single-dose formulation refers to a unit dose formulation, wherein the compound represented by formula (I) or the salt thereof is loaded within 1 unit package.

According to an embodiment of the present disclosure, the medicament or the pharmaceutical composition may further comprise a pharmaceutically acceptable auxiliary material, such as a carrier or an excipient. The pharmaceutically acceptable auxiliary material is preferably chemically unreactive or inert to the active ingredient. For example, the pharmaceutically acceptable auxiliary material is selected from at least one of the following auxiliary materials, including but not limited to, fillers, disintegrants, adhesives, lubricants, surfactants, flavoring agents, wetting agents, matrixes, and the like.

According to an embodiment of the present disclosure, the route of administration of the medicament or the pharmaceutical composition includes, but is not limited to, gastrointestinal administration or parenteral administration, wherein the gastrointestinal administration may be oral administration; the parenteral administration may be topical administration, transdermal administration, injection, and the like.

In one embodiment, the route of administration of the medicament or the pharmaceutical composition may be topical administration in combination with oral administration.

According to an embodiment of the present disclosure, the dosage form of the medicament or the pharmaceutical composition may be selected from a capsule, a tablet, a patch, a film, a pill, a powder, a lozenge, a sachet, a cachet, an elixir, a suspension, an emulsion, a solution formulation, a syrup, an aerosol, an ointment, a cream, a suppository, or an injection.

### Definitions and Description

Unless otherwise stated, the definitions of terms described in the specification and claims of the present application, including definitions thereof as examples, exemplary definitions, preferred definitions, definitions documented in tables, definitions of specific compounds in the examples, and the like, may be arbitrarily combined and incorporated with each other. Such combinations and incorporation shall fall within the scope of the present specification.

The term "effective amount" or "therapeutically effective amount" refers to an amount of the compounds of the present disclosure sufficient to effect the intended use, including, but not limited to, the treatment of a disease as defined below. The therapeutically effective amount may vary depending on the following factors: the intended use *(in vitro* or *in vivo),* or the subject and diseases or conditions being treated, such as the body weight and age of the subject, severity of the diseases or conditions, and route of administration, which may be readily determined by one of ordinary skill in the art. The specific dosage will vary depending on the following factors: the selected particular compound, the dosage regimen to be followed, whether to administer in combination with other compounds, the schedule of administration, the tissue to be administered, and the physical delivery system carried.

The term "patient" refers to a mammal suffering from or at risk of suffering from the ulcer, for example, the patient is selected from rodents, cows, pigs, dogs, cats, and primates, particularly humans.

### Advantageous Effects

In the present disclosure, it is unexpectedly found that the compound represented by formula (I) or the pharmaceutically acceptable salt thereof can effectively prevent or treat ulcers, and particularly, has an excellent therapeutic effect on oral ulcers.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure.

Unless otherwise stated, the starting materials and reagents used in the following examples are all commercially available products or can be prepared by using known methods.

### Example 1: Assay for Therapeutic Effect on Oral Ulcer

In the following experiment, the selected medicament was a cream containing 1% tapinarof, in which 1% tapinarof by mass percentage, as well as mometasone furoate, propylene glycol, white vaseline, light liquid paraffin, hexadecanol, glyceryl monostearate, purified water, ethylparaben, and the like, were contained.

The administration method comprises the following steps: squeezing a small amount of the cream described above and applying it on a cotton swab; after the oral hygiene was performed following three meals and before sleep, applying it to cover the surface of the ulcer, 4 times daily; and continuing the administration until the ulcer was healed. No other medicaments were used during the administration.

Subject 1 suffered from non-recurrent oral ulcers, with multiple ulcers in the oral cavity, about 0.3 × 0.6 cm, the course of the disease was about 10 days, and no other medicaments were used. After the administration, the pain was significantly relieved on day 1, the ulcers were significantly improved on day 2, and the ulcers were completely healed and disappeared on day 4.

Subject 2 suffered from recurrent oral ulcers, with multiple ulcers in the oral cavity, about 0.3 × 0.7 cm, the course of the disease was about 6 days, and no other medicaments were used. After the administration, the pain was significantly relieved on day 1, and the ulcers were completely healed and disappeared on day 3.

### Example 2: Efficacy in SD Male Rat Oral Ulcer Model After 7 Days of Treatment

Eighteen SD male rats were randomly divided into 3 groups, i.e., a model group (propylene glycol vehicle group), a 2% indigo group (1 mg/rat), and a 2% tapinarof group (1 mg/rat). Oral ulcer modeling: a 1 cm × 0.6 cm filter paper (soaked into a 60% glacial acetic acid solution) was placed on the labial mucosa of the alveolar bone beneath the lower incisor in a rat and maintained for 1 min. Administration: after the rats were anesthetized, 50 µL of the solution in each group was pipetted by using a pipettor and evenly applied to the ulcer part 2 times daily for 7 consecutive days. The experimental results are shown in Table 1. After each group was treated with glacial acetic acid, symptoms such as pseudomembrane covering, central depression, red and swollen edges, significant pain response after stimulation, etc. appeared, and after the administration was completed, various symptoms were relieved. 2% tapinarof had a good therapeutic effect, and its efficacy was superior to that of 2% indigo.

**Table 1. Efficacy in SD male rat oral ulcer model after 7 days of treatment (n = 6)**

| Group | Ulcer area (mm²) | | Ulcer healing rate |
|---|---|---|---|
| | Day 0 | Day 7 | |
| Vehicle | 47.27 | 37.46 | 21% |
| 2% indigo | 46.33 | 22.09 | 52% |
| 2% tapinarof | 46.90 | 19.70 | 58% |

| | | | |
|---|---|---|---|
| Note: Ulcer healing rate = (ulcer area on Day 0 - ulcer area on Day 10)/ulcer area on Day 0 × 100% | | | |

### Example 3: Efficacy in DB/DB Male Mouse Diabetic Foot Ulcer Model After 10 Days of Treatment

Eighteen DB/DB male mice were randomly divided into 3 groups, i.e., a model group (propylene glycol vehicle group), a 2% indigo group (1 mg/mouse), and a 2% tapinarof group (1 mg/mouse). Foot ulcer modeling: after the mouse was anesthetized, the foot was disinfected; a 4 mm × 5 mm rectangular region was marked on the dorsum of the foot; and the skin and fascia were removed within the region.

Administration: on the dorsum of the foot of the mouse, 50 µL of the solution in each group was pipetted by using a pipettor and evenly applied to the ulcer part 2 times daily for 10 consecutive days, and the length and the width of the ulcer were recorded.

The experimental results are shown in Table 2. After the mice in each group were treated, the feet were red and swollen, ulcerations appeared around the wound, the pain was significantly reflected, and the mice had paw withdrawal behaviors. After 10 days of administration, 2% tapinarof had a significant inhibitory effect, which was superior to that of the 2% indigo group

**Table 2. Efficacy in DB/DB male mouse diabetic foor ulcer model after 10 days of treatment (n = 6)**

| Group | Ulcer area (mm²) | | (n = 6) Ulcer healing rate |
|---|---|---|---|
| | Day 0 | Day 10 | |
| Vehicle | 17.64 | 16.70 | 5% |
| 2% indigo | 17.99 | 13.30 | 26% |
| 2% tapinarof | 18.19 | 10.93 | 39%** |

| | | | |
|---|---|---|---|
| Note: Ulcer healing rate = (ulcer area on Day 0 - ulcer area on Day 10)/ulcer area on Day 0 × 100% Statistical analysis was performed by using one-way ANOVA. As compared to the vehicle group, ** indicates p < 0.05. | | | |

The embodiments of the technical solutions of the present disclosure have been described above by way of example. It will be appreciated that the claimed scope of the present disclosure is not limited to the embodiments described above. Any modification, equivalent substitution, improvement, and the like made by those skilled in the art without departing from the spirit and principle of the present disclosure shall fall within the claimed scope of the present application.

## Claims

1. Use of at least one of a compound represented by formula (I) below and a pharmaceutically acceptable salt thereof for manufacturing a medicament for the prevention and/or treatment of an ulcer:

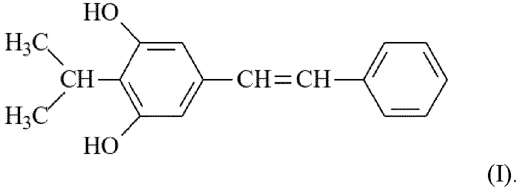

2. The use according to claim 1, wherein the medicament is a pharmaceutical composition; preferably, the pharmaceutical composition comprises at least one of the compound represented by formula (I) and the pharmaceutically acceptable salt thereof described above; and
more preferably, the pharmaceutical composition comprises a therapeutically effective amount of at least one of the compound represented by formula (I) and the pharmaceutically acceptable salt thereof.

3. The use according to claim 1 or 2, wherein the compound represented by formula (I) is an E or Z isomer, preferably an E isomer represented by formula (I-1) below:

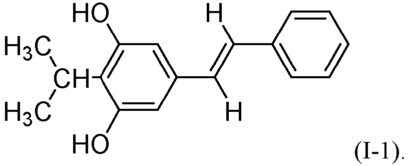

4. The use according to any one of claims 1-3, wherein the ulcer is selected from one or more of a pressure ulcer, a genital ulcer, ulcerative dermatitis, an anal ulcer, a rectal ulcer, a foot ulcer, a diabetic foot ulcer, a corneal ulcer, an oral ulcer, an aphthous ulcer, a peptic ulcer, a venous ulcer, a stress ulcer, ulcerative sarcoidosis, ulcerative lichen planus, ulcerative colitis, and ulcerative disposition.

5. The use according to claim 4, wherein the oral ulcer is selected from a recurrent oral ulcer, a traumatic oral ulcer, and an oral ulcer associated with or caused by a disease.

6. The use according to claim 5, wherein the recurrent oral ulcer is selected from a recurrent aphthous ulcer or a frequent oral ulcer.

7. The use according to claim 5, wherein the traumatic oral ulcer comprises oral ulcers caused by a mechanical injury, a chemical burn, and cold and hot stimulation.

8. The use according to claim 5, wherein the oral ulcer associated with or caused by a disease comprises oral ulcers associated with or caused by diseases such as Behcet's disease, Reiter's syndrome, a tumor, etc.

9. The use according to claim 5, wherein the oral ulcer is selected from at least one of the recurrent oral ulcer and the traumatic oral ulcer. The use according to any one of claims 1-9, wherein the medicament is a single-dose formulation; preferably, in the single-dose formulation, the content of the compound represented by formula (I) and the pharmaceutically acceptable salt thereof is a therapeutically effective amount, such as 1-10 mg, based on the compound represented by formula (I).
